(19) Europäisches Patentamt
European Patent Office
Office européen des brevets

(11) EP 1 133 975 B1

(12) **EUROPEAN PATENT SPECIFICATION**

(45) Date of publication and mention of the grant of the patent:
**13.02.2008 Bulletin 2008/07**

(51) Int Cl.:
***A61Q 5/10*** *(2006.01)* ***A61K 8/41*** *(2006.01)*

(21) Application number: **01106318.7**

(22) Date of filing: **15.03.2001**

(54) **Use of a compound for dyeing hair**

Verwendung einer Verbindung zum Haare färben

Utilisation d'un composé pour la coloration capillaire

(84) Designated Contracting States:
**DE FR GB**

(30) Priority:

**17.03.2000 JP 2000076009**
**17.03.2000 JP 2000076637**
**17.03.2000 JP 2000076638**
**17.03.2000 JP 2000076668**
**07.04.2000 JP 2000107178**
**07.04.2000 JP 2000107180**
**07.04.2000 JP 2000107187**
**07.04.2000 JP 2000107188**
**07.04.2000 JP 2000107189**
**07.04.2000 JP 2000107190**
**27.06.2000 JP 2000193177**
**27.06.2000 JP 2000193178**
**27.06.2000 JP 2000193180**
**27.06.2000 JP 2000193181**
**27.06.2000 JP 2000193184**
**27.06.2000 JP 2000193188**

(43) Date of publication of application:
**19.09.2001 Bulletin 2001/38**

(73) Proprietor: **KAO CORPORATION**
**Tokyo 103-8210 (JP)**

(72) Inventors:

- **Ohashi, Yukihiro**
**Sumida-ku,**
**Tokyo 131-8501 (JP)**
- **Miyabe, Hajime**
**Sumida-ku,**
**Tokyo 131-8501 (JP)**
- **Matsunaga, Kenichi**
**Sumida-ku,**
**Tokyo 131-8501 (JP)**
- **Totoki, Shintaro**
**Sumida-ku,**
**Tokyo 131-8501 (JP)**
- **Saito, Yoshinori**
**Sumida-ku,**
**Tokyo 131-8501 (JP)**

(74) Representative: **HOFFMANN EITLE**
**Patent- und Rechtsanwälte**
**Arabellastrasse 4**
**81925 München (DE)**

(56) References cited:
**WO-A-95/01772** **FR-A- 2 757 388**

- **DATABASE CAPLUS [Online] CHEMICAL ABSTRACTS SERVICE, COLUMBUS, OHIO, US; 1979, XP002259111 accession no. STN Database accession no. 90:205784 & JP 54 008626 A (HODOGAYA CHEMICAL CO.) 23 January 1979 (1979-01-23)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the use of a specified compound for dyeing hair featuring markedly high hair dyeing power, less color fade over time and less color tone change of the dye after storage.

## Background Art

Hair dyes can be classified by the dye to be used therefor, or whether they have bleaching action of melanin or not. Typical examples include a two-part permanent hair dye composed of a first part containing an alkali agent, an oxidation dye and a direct dye such as nitro dye and a second part containing an oxidizing agent; and one-part semi-permanent hair dye containing an organic acid or an alkali agent, and a direct dye such as acid dye, basic dye or nitro dye.

The above-described permanent hair dye is however accompanied with the drawbacks that color tone imparted by an oxidation dye is not so vivid and the color of the hair dyed with a vivid-color producing nitro dye ordinarily employed as a direct dye markedly fades over time and becomes dull soon even if the color tone rightly after dyeing is very vivid (Japanese Patent Application Laid-Open (*Kokai*) No. Hei 6-271435).

Recently, hair dyes containing as a direct dye a so-called cationic dye having a cation group contained in their conjugate system have been reported (Japanese Language Laid-Open Publication (PCT) No. Hei 8-507545, 8-501322 or 10-502946, or Japanese Patent Application Laid-Open (*Kokai*) No. Hei 10-194942). They have been found to involve drawbacks that intended dyeing effects are not available owing to decomposition of them caused by mixing, upon hair dyeing, with hydrogen peroxide ordinarily employed as an oxidizing agent; and that they are unstable to an alkali agent or a reducing agent essentially contained in a permanent hair dye.

## Disclosure of the Invention

An object of the present invention is therefore to provide the use, for dyeing hair, of a compound featuring high hair dyeing power, less color fade over time and less color tone change of the dye upon storage owing to excellent storage stability.

The present inventors have found that by using a compound represented by the below-described formula (I) which is known as a dye for dyeing or printing fibrous materials, for dyeing hair the composition containing said compounds is free from decomposition of the dye upon hair dyeing, has markedly high hair dyeing power, exhibits excellent light resistance, washing resistance, perspiration resistance, friction resistance and heat resistance, and causes a less change in the color tone of the dye after storage as compared with that rightly after preparation because the dye exists in the composition stably.

In one aspect of the present invention, there is thus provided the use of a compound represented by the below-described formulas (I) for dyeing hair,

<General formula (I)>

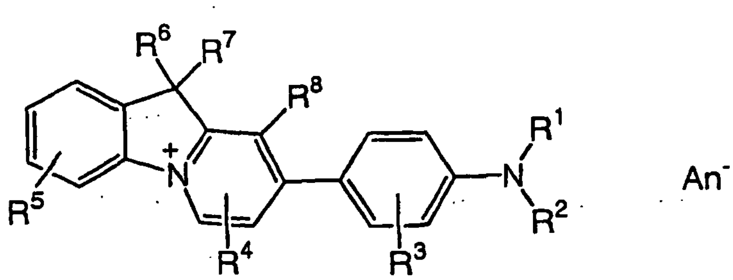

(I)

[in the formula (I), $R^1$ represents a hydrogen atom, a $C_{1-3}$ alkyl group which may have a substituent or an aralkyl group,

$R^2$ represents a $C_{1-3}$ alkyl group which may have a substituent, an aralkyl group or a phenyl group which may have a substituent,
$R^3$ represents a hydrogen atom, a halogen atom, a $C_{1-3}$ alkyl group or a $C_{1-3}$ alkoxy group,
$R^4$ represents a $C_{1-3}$ alkyl group or a phenyl group which may have a substituent,
$R^5$ represents a hydrogen atom, a halogen atom, a $C_{1-3}$ alkyl group or a $C_{1-3}$ alkoxy group,

$R^6$ and $R^7$ are the same or different and each independently represents a $C_{1-3}$ alkyl group,
$R^8$ represents a hydrogen atom, a methyl group or a cyano group, and
$An^-$ stands for an anion.]

**Best Modes for Carrying Out the Invention**

<Direct Dye (I)>

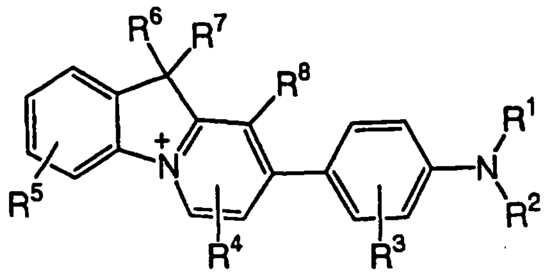

An$^-$ (I)

Direct dye (I) is a cationic dye (Japanese Patent Application Laid-Open (Kokai) No. Sho 54-8626) which is known to dye therewith polyacrylonitrile fibers or polyester or polyamide fibers having, as a dyeing seat, an acid residue and it can impart the hair with a strong fluorescent color ranging from greenish yellow to reddish yellow at a markedly high density.
In the formula (I), examples of $R^1$ include hydrogen atom and methyl, ethyl, 2-cyanoethyl, 2-chloroethyl, 2-methoxyethyl, 2-hydroxyethyl and benzyl groups.
In the formula (I), examples of $R^2$ include methyl, ethyl, 2-cyanoethyl, 2-chloroethyl, 2-methoxyethyl, 2-hydroxyethyl, benzyl, phenyl, p-tolyl, p-chlorophenyl and p-methoxyphenyl groups.
In the formula (I), examples of $R^3$ include hydrogen atom, chlorine atom, methyl group and methoxy group.
In the formula (I), examples of $R^4$ include methyl, ethyl, phenyl, p-tolyl, p-chlorophenyl and p-methoxyphenyl groups.
In the formula (I), examples of $R^5$ include hydrogen atom, chlorine atom, bromine atom, methyl group, ethyl group, methoxy group and ethoxy group.
In the formula (I), examples of $R^6$ or $R^7$ include methyl and ethyl groups.
In the formula (I), examples of the anion represented by $An^-$ include chloride ions, bromide ions, iodide ions, trichlorozincic acid ions, tetrachlorozincic acid ions, sulfuric acid ions, hydrogensulfuric acid ions, methyl sulfate ions, phosphoric acid ions, formic acid ions and acetic acid ions.
The followings are the specific examples of the direct dye (I) usable in the present invention.

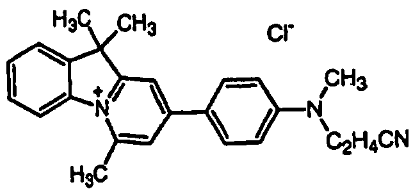
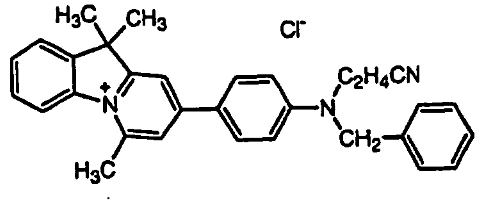
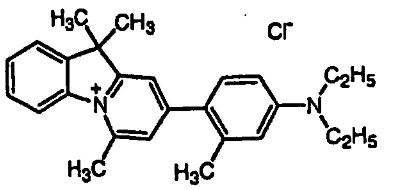
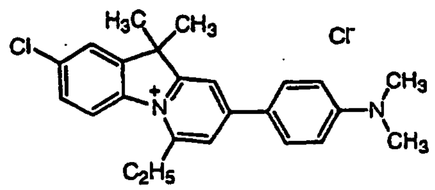
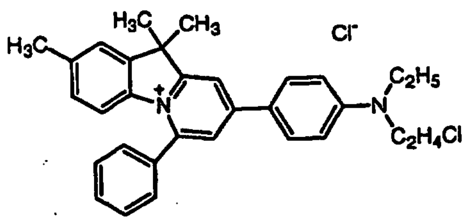
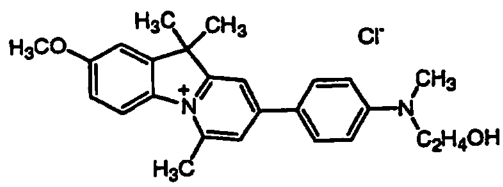
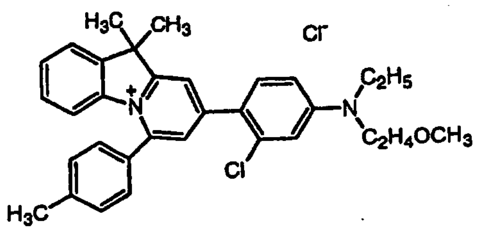
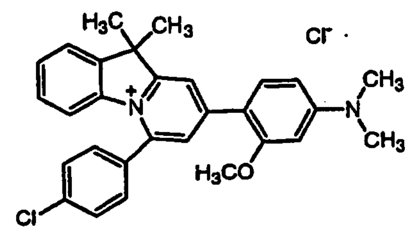
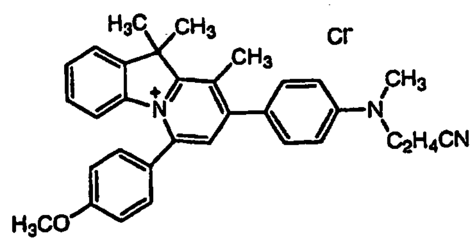
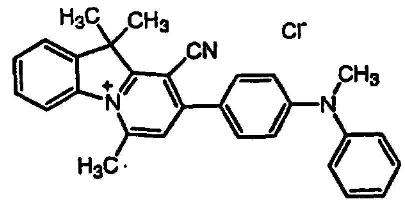
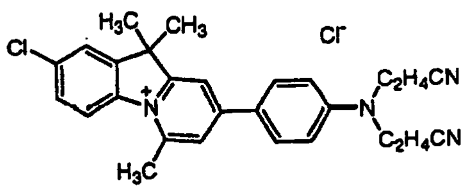
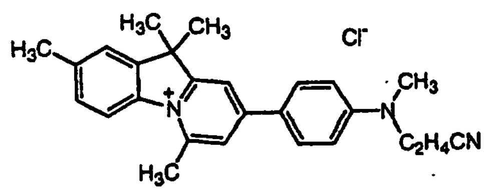

One or more direct dyes can be used as the direct dye (I). The direct dye (I) can also be used in combination with another direct dye. In particular, combined use with a red or blue dye makes it possible to dye the hair with deep and very lustrous dark brown or black color.

Examples of the direct dye other than the direct dyes (I) include Basic Blue 7 (C.I. 42595), Basic Blue 26 (C.I. 44045), Basic Blue 99 (C.I. 56059), Basic Violet 10 (C.I. 45170), Basic Violet 14 (C.I. 42515), Basic Brown 16 (C.I. 12250), Basic Brown 17 (C.I. 12251), Basic Red 2 (C.I. 50240), Basic Red 22 (C.I. 11055), Basic Red 76 (C.I. 12245), Basic Red 118 (C.I. 12251:1) and Basic Yellow 57 (C.I. 12719); and basic dyes as described in Japanese Patent Publication No. Sho 58-2204, Japanese Patent Application Laid-Open (Kokai) No. Hei 9-118832, Japanese Language Laid-Open Publication (PCT) No. Hei 8-501322 and Japanese Language Laid-Open Publication (PCT) No. Hei 8-507545.

The content of each of the direct dye (I) is preferably 0.01 to 20 wt.%, more preferably 0.05 to 10 wt.%, especially 0.1 to 5 wt.% in the whole composition (after mixing in the case of a two-part or three-part type composition. This will apply equally hereinafter). When another direct dye is used in combination, the total content of it with each of the direct

dye. (I) is preferably 0.05 to 10 wt.%, especially 0.1 to 5 wt.%.

The hair dye composition of the present invention is preferably adjusted to pH 6 to 11, especially 8 to 11. Examples of the alkali agent for adjusting pH include ordinarily employed ones such as ammonia and organic amine, and salts thereof. The alkali agent is added preferably in an amount of 0.01 o 20 wt.%, more preferably 0.1 to 10 wt.%, especially 0.5 to 5 wt.% in the whole composition.

To the hair dye composition of the present invention, an oxidizing agent can be added. In this case, bleaching can be effected simultaneously with dyeing, which enables more vivid hair dyeing. Examples of the oxidizing agent include ordinarily employed ones such as hydrogen peroxide, persulfates such as ammonium persulfate, potassium persulfate and sodium persulfate; perborates such as sodium perborate; percarbonates such as sodium percarbonate and bromates such as sodium bromate and potassium bromate, with hydrogen peroxide being particularly preferred. The oxidizing agent is added in an amount of 0.5 to 10 wt.%, especially 1 to 8 wt.% in the whole composition.

In the hair dye composition of the present invention, an oxidation dye can be incorporated further. This incorporation enables remarkable vivid dyeing not attainable by the single use of an oxidation dye. When an oxidation dye is incorporated, the above-exemplified oxidizing agents, preferably hydrogen peroxide is employed. Alternatively, an oxidizing enzyme such as laccase can be employed. For the oxidation dye, known color developers and couplers ordinarily employed for an oxidation type hair dye can be used.

Examples of the developer include p-phenylenediamines having one or several groups selected from $NH_2$- , NHR- and $NR_2$- groups (in which, R represents a $C_{1-4}$ alkyl or hydroxyalkyl group) such as p-phenylenediamine, p-toluylenediamine, N-methyl-p-phenylenediamine, chloro-p-phenylenediamine, 2-(2'-hydroxyethylamino)-5-aminotoluene, N,N-bis-(2-hydroxyethyl)-p-phenylenediamine, 2-hydroxyethyl-p-phenylenediamine, 2,6-dimethyl-p-phenylenediamine, methoxy-p-phenylenediamine, 2,6-dichloro-p-phenylenediamine, 2-chloro-6-methyl-p-phenylenediamine, 6-methoxy-3-methyl-p-phenylenediamine, 2,5-diaminoanisole, N-(2-hydroxypropyl)-p-phenylenediamine and N-2-methoxyethyl-p-phenylenediamine; 2,5-diaminopyridine derivatives and 4,5-diaminopyrazole derivatives; p-aminophenols such as p-aminophenol, 2-methyl-4-aminophenol, N-methyl-p-aminophenol, 3-methyl-4-aminophenol, 2,6-dimethyl-4-aminophenol, 3,5-dimethyl-4-aminophenol, 2,3-dimethyl-4-aminophenol and 2,5-dimethyl-4-aminophenol; o-aminophenols, o-phenylenediamines, 4,4'-diaminophenylamine and hydroxypropylbis(N-hydroxyethyl-p-phenylenediamine); and salts thereof.

Examples of the coupler include 1-naphthol, 1,5-dihydroxynaphthalene, 1,7-dihydroxynaphthalene, 2,7-dihydroxynaphthalene, 5-amino-2-methylphenol, 5-(2'-hydroxyethylamino)-2-methylphenol, 2,4-diaminoanisole, m-toluylenediamine, resorcin, m-phenylenediamine, m-aminophenol, 4-chlororesorcin, 2-methylresorcin, 2,4-diaminophenoxyethanol, 2,6-diaminopyridine, 2-amino-3-hydroxypyridine, 4-hydroxyindole, 6-hydroxyindole, 2,4-diamino-6-hydroxypyrimidine, 2,4,6-triaminopyrimidine, 2-amino-4,6-dihydroxypyrimidine, 4-amino-2,6-dihydroxypyrimidine, 4,6-diamino-2-hydroxypyrimidine and 1,3-bis(2,4-diaminophenoxy)propane; and salts thereof.

As a developer or coupler, at least one of the above-exemplified ones can be used. It is preferably added in an amount of 0.01 to 20 wt.%, especially 0.5 to 10 wt.% based on the whole composition.

To the hair dye composition of the present invention, a known autoxidation dye typified by an indole or an indoline, or a known direct dye such as a nitro dye or a disperse dye can also be added.

When an anionic component (such as anionic surfactant or anionic polymer) is added to the hair dye composition of the present invention in the case where the direct dye (I) are cationic ones, it is preferred to satisfy the following equation:

"Ion activity concentration of an anionic component/ion activity concentration of a cationic direct dye (I) ≤ 8"

The term "Ion activity concentration" as used herein means "molar concentration x ionic valence"

Addition of a polyol, polyol alkyl ether, cationic or amphoteric polymer or silicone to the hair dye composition of the present invention is preferred, because the resulting composition can dye the hair uniformly and has improved cosmetic effects.

In addition to the above-described components, those ordinarily employed as a raw material for cosmetics can be added to the hair dye composition of the present invention. Examples of such an optional component include hydrocarbons, animal or vegetable fats and oils, higher fatty acids, organic solvents, penetration promoters, cationic surfactants, natural or synthetic polymers, higher alcohols, ethers, amphoteric surfactants, nonionic surfactants, protein derivatives, amino acids, antiseptics, chelating agents, stabilizing agents, antioxidants, plant extracts, crude drug extracts, vitamins, colorants, perfumes and ultraviolet absorbers.

The hair dye composition of the present invention can be prepared in a conventional manner into a one-part composition, a two-part composition having a first-part component containing an alkali agent and a second-part component containing an oxidizing agent, or a three-part composition having, in addition to these two components, a powdery oxidizing agent such as persulfate. The direct dye (I) can each be incorporated in either one or both of these components of the two-part or three-part composition. The one-part type is applied to the hair directly, while the two- or three-part type is applied to the hair after mixing these parts upon hair dyeing.

No particular limitation is imposed on the form of the hair dye composition of the present invention. Examples include powder, transparent liquid, emulsion, cream, gel, paste, aerosol, and aerosol foam. It preferably has a viscosity of 2000 to 100000 mPa·s in the stage of application to the hair (after mixing of all the components when a two-part or three-part type composition is employed).

-Examples-

<Direct dye (I)>

Examples of the direct dye (I) will next be described. Compounds used in these examples are as follows:

Compound (Ia)

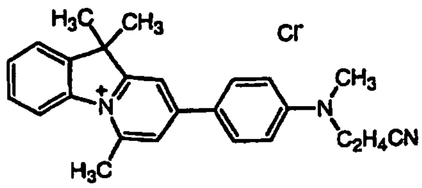

Compound (Ib)

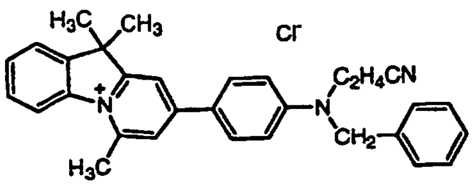

Compound (Ic)

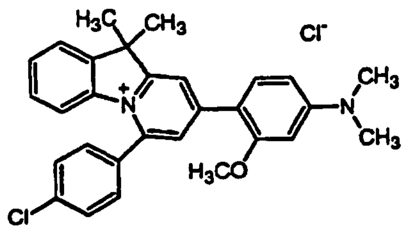

Compound (α)

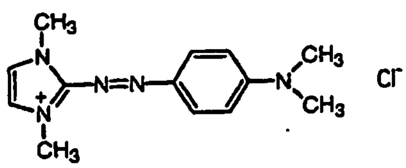

Examples I-1 to I-12

In a manner known <u>per se</u> in the art, hair dyes as shown in Tables I-1 to I-3 were prepared.

Table I-1

| (wt.%) | Examples | | | | |
|---|---|---|---|---|---|
| | I-1 | I-2 | I-3 | I-4 | I-5 |
| Dye [Compound (Ia)] | 0.2 | | 0.15 | 0.1 | |
| Dye [Compound (Ib)] | | 0.5 | | 0.1 | 0.2 |
| Dye [Compound (α)] | | | 0.15 | 0.1 | 0.05 |
| Dye [Basic Blue 7] | | | 0.1 | 0.1 | |
| Ethanol | | 5 | | 5 | 5 |
| Propylene glycol | | | 5 | | 5 |
| Diethylene glycol monoethyl ether | | 10 | | | |
| Guar gum | 1 | | | | |
| Hydroxypropyl guar gum | | 1 | 1 | 1 | 1 |
| "Gufquat 734" (trade name; product of ISP Japan) | 1 | | 1 | | |
| "Catinal LC100" (trade name; product of Toho Chemical Industry) | | 1 | | | 1 |
| "Polyether-modified silicone KF6005" trade name; (product of Shin-Etsu Chemical) | | | | | 0.4 |
| "Amodimethicone SM8702C" (product of Dow Corning Toray Silicone) | | | | 1.5 | |
| Monoethanolamine | 0.1 | | | | |
| Phosphoric acid | Amount to adjust pH to 9 | | | | |
| Perfume | q.s. | | | | |
| Water | Balance | | | | |
| Total (g) | 100 | | | | |

Table 1-2

| (wt.%) | | Examples | | | |
|---|---|---|---|---|---|
| | | I-6 | I-7 | I-8 | I-9 |
| 1st part | Dye (Compound (la)] | 0.2 | | 0.15 | 0.2 |
| | Dye [Compound (lb)] | | 0.1 | 0.15 | |
| | Dye [Compound (α)] | | 0.1 | | 0.05 |
| | Dye [Basic Blue 99] | | 0.3 | | |
| | 28 wt.% aqueous ammonia | 5 | | | |
| | Monoethanolamine | 2 | | | |
| | Propylene glycol | 8 | | | |
| | Polyoxyethylene (20) isostearyl ether | 24 | | | |
| | Polyoxyethylene (2) isostearyl ether | 20 | | | |
| | "Merquat 280' (trade name; product of Calgon Corp., 35 wt.% aqueous solution) | 8 | | | |
| | "Polymer JR400° (trade name; product of Union Carbide) | | 0.5 | | 0.5 |
| | "Amodimethicone SM8702C" (trade name; product of Dow Corning Toray Silicone) | | | 2 | |
| | "Polyether modified silicone KF6005" (trade name; product of Shin-Etsu Chemical) | | | | 0.3 |
| | Tetrasodium ethylenediaminetetraacetate | 0.1 | | | |
| | Perfume | q.s. | | | |
| | Ammonium chloride | Amount to adjust pH to 10 | | | |
| | Water | Balance | | | |
| 2nd part | 35 wt.% aqueous hydrogen peroxide | 17.1 | | | |
| | Methylparaben | 0.1 | | | |
| | Phosphoric acid | Amount to adjust pH to 3.5 | | | |
| | Water | Balance | | | |

Table 1-3

| (wt.%) | | Examples | | |
|---|---|---|---|---|
| | | 1-10 | 1-11 | 1-12 |
| 1st part | Toluene-2,5-diamine | 1.9 | 1 | |
| | Para-aminophenol | | | 1 |
| | Resorcin | 2 | | |
| | Para-amino-ortho-cresol | | | 1.1 |
| | 2,4-Diaminophenoxyethanol | | 1.37 | |
| | Dye [Compound (Ia)] | 0.05 | | |
| | Dye [Compound (Ib)] | | 0.15 | |
| | Dye [Compound (Ic)] | | | 0.1 |
| | 28 wt.% aqueous ammonia | 5 | | |
| | Monoethanolamine | 2 | | |
| | Propylene glycol | 8 | | |
| | Polyoxyethylene (20) isostearyl ether | 24 | | |
| | Polyoxyethylene (2) isostearyl ether | 20 | | |
| | "Merquat 280" (trade name; product of Calgon Corp., a 35 wt.% aqueous solution) | 8 | | |
| | "Polymer JR400" (product of Union Carbide) | | 0.5 | |
| | "Amodimethicone SM8702C" (trade name; product of Dow Corning Toray Silicone) | | | 2 |
| | Sodium sulfite | 0.05 | | |
| | Ascorbic acid | 0.5 | | |
| | Tetrasodium ethylenediaminetetraacetate | 0.1 | | |
| | Perfume | q.s. | | |
| | Ammonium chloride | Amount to adjust the pH to 10 | | |
| | Water | Balance | | |
| 2nd part | 35 wt.% Aqueous hydrogen peroxide | 17.1 | | |
| | Methylparaben | 0.1 | | |
| | Phosphoric acid | Amount to adjust the pH to 3.5 | | |
| | Water | Balance | | |

Examples I-13

In a manner known per se in the art, the below-described hair dye was prepared.

| First part | (wt.%) |
|---|---|
| Para-aminophenol | 1 |
| Para-amino-ortho-cresol | 1.1 |
| Compound (Ic) | 0.1 |
| 28 wt.% aqueous ammonia | 5 |
| Monoethanolamine | 2 |
| Cetanol | 8.5 |
| Polyoxyethylene (40) cetyl ether | 3 |

(continued)

| First part | (wt.%) |
|---|---|
| Polyoxyethylene (2) cetyl ether | 3.5 |
| Stearyl trimethylammonium chloride | 2 |
| Liquid paraffin | 0.5 |
| Sodium sulfite | 0.05 |
| Ascorbic acid | 0.5 |
| Tetrasodium ethylenediaminetetraacetate | 0.1 |
| Perfume | q.s. |
| Ammonium chloride | Amount to adjust pH to 10 |
| Water | Balance |
| Second part | (wt.%) |
| 35 wt.% aqueous hydrogen peroxide | 17.1 |
| Methylparaben | 0.1 |
| Phosphoric acid Amount | to adjust pH 3.5 |
| Water | Balance |

**Claims**

1. Use of a compound represented by the following formula (I) :

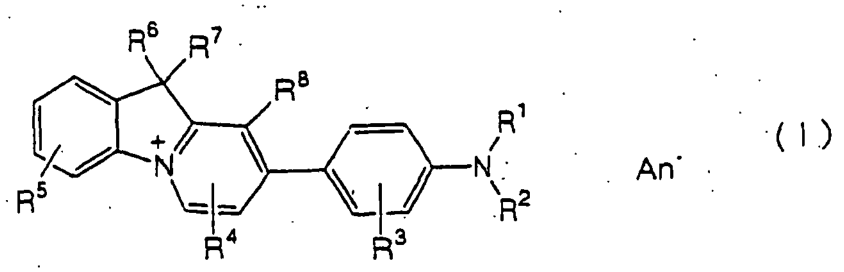

in the formula (I), $R^1$ represents a hydrogen atom, a $C_{1-3}$ alkyl group which may have a substituent or an aralkyl group.

$R^2$ represents a $C_{1-3}$ alkyl group which may have a substituent, an aralkyl group or a phenyl group which may have a substituent,
$R^3$ represents a hydrogen atom, a halogen atom, a $C_{1-3}$ alkyl group or a $C_{1-3}$ alkoxy group,
$R^4$ represents a $C_{1-3}$ alkyl group or a phenyl group which may have a substituent,
$R^5$ represents a hydrogen atom, a halogen atom, a $C_{1-3}$ alkyl group or a $C_{1-3}$ alkoxy group,
$R^6$ and $R^7$ are the same or different and each independently represents a $C_{1-3}$ alkyl group,
$R^8$ represents a hydrogen atom, a methyl group or a cyano group, and
$An^-$ stands for an anion for dyeing hair.

2. Use according to claim 1, wherein the composition further comprises an oxidizing agent.

3. Use according to claim 1 or 2, wherein the composition further comprises an oxidation dye.

**Patentansprüche**

1. Verwendung einer Verbindung mit der folgenden Formel (I):

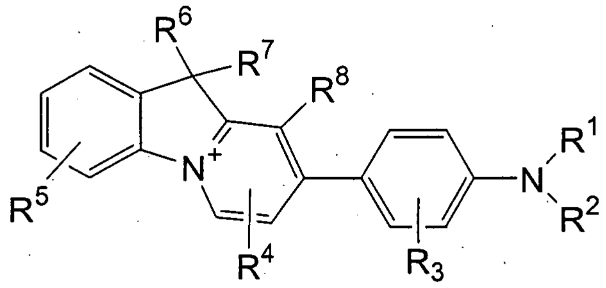

An⁻ (I)

worin in der Formel (I)

$R^1$ ein Wasserstoffatom, eine $C_{1-3}$-Alkylgruppe, die einen Substituenten haben kann, oder eine Aralkylgruppe ist,
$R^2$ eine $C_{1-3}$-Alkylgruppe, die einen Substituenten haben kann, eine Aralkylgruppe oder Phenylgruppe ist, die einen Substituenten haben kann,
$R^3$ ein Wasserstoffatom, Halogenatom, eine $C_{1-3}$-Alkylgruppe oder $C_{1-3}$-Alkoxygruppe ist,
$R^4$ eine $C_{1-3}$-Alkylgruppe oder Phenylgruppe ist, die einen Substituenten haben kann,
$R^5$ ein Wasserstoffatom, Halogenatom, eine $C_{1-3}$-Alkylgruppe oder $C_{1-3}$-Alkoxygruppe ist,
$R^6$ und $R^7$ gleich oder verschieden sind und jeweils unabhängig eine $C_{1-3}$-Alkylgruppe bedeuten,
$R^8$ ein Wasserstoffatom, eine Methylgruppe oder Cyanogruppe ist und
$An^-$ für ein Anion steht,

zum Färben von Haar.

2. Verwendung nach Anspruch 1, worin die Zusammensetzung weiterhin ein Oxidationsmittel enthält.

3. Verwendung nach Anspruch 1 oder 2, worin die Zusammensetzung weiterhin einen Oxidationsfarbstoff enthält.

**Revendications**

1. Utilisation d'un composé représenté par la formule (I) suivante :

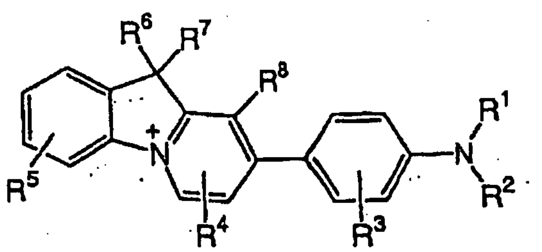

An⁻ (I)

dans la formule (I), $R^1$ représente un atome d'hydrogène, un groupe $C_{1-3}$ alkyle qui peut avoir un substituant ou un groupe aralkyle,

$R^2$ représente un groupe $C_{1-3}$ alkyle qui peut avoir un substituant, un groupe aralkyle ou un groupe phényle qui peut avoir un substituant,
$R^3$ représente un atome d'hydrogène, un atome d'halogène, un groupe $C_{1-3}$ alkyle ou un groupe $C_{1-3}$ alcoxy,
$R^4$ représente un groupe $C_{1-3}$ alkyle ou un groupe phényle qui peut avoir un substituant,
$R^5$ représente un atome d'hydrogène, un atome d'halogène, un groupe $C_{1-3}$ alkyle ou un groupe $C_{1-3}$ alcoxy,
$R^6$ et $R^7$ sont identiques ou différents et représentent chacun indépendamment un groupe $C_{1-3}$ alkyle,
$R^8$ représente un atome d'hydrogène, un groupe méthyle ou un groupe cyano, et
$An^-$ représente un anion

pour la teinture des cheveux.

2. Utilisation selon la revendication 1 où la composition comprend en outre un agent oxydant.

3. Utilisation selon la revendication 1 ou 2 où la composition comprend en outre un colorant d’oxydation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6271435 A
- JP 8507545 A
- JP 8501322 A
- JP 10502946 A
- JP 10194942 A
- JP 54008626 A
- JP 58002204 A
- JP 9118832 A